# EUROPÄISCHE PATENTSCHRIFT

(11) **EP 1 247 792 B1**
(45) Veröffentlichungstag und Bekanntmachung des Hinweises auf die Patenterteilung: **09.08.2006**
(21) Anmeldenummer: 02006615.5
(22) Anmeldetag: 25.03.2002
(51) Int. Cl.: C07C 29/147, C07C 33/46, C07C 51/08, C07C 63/26

(54) **Verfahren zur Herstellung von halogensubstituierten Dibenzylalkoholen**
Process for the preparation of halo-substituted dibenzyl alcohols
Procédé de préparation d'alcools dibenzyliques halogengés

(30) Priorität: 06.04.2001 DE 10117206
(43) Veröffentlichungstag der Anmeldung: 09.10.2002
(73) Patentinhaber: LANXESS Deutschland GmbH, 51369 Leverkusen (DE)
(72) Erfinder: Rodefeld, Lars, Dr., 51371 Leverkusen (DE)

(56) Entgegenhaltungen:
- EP-A- 0 302 612
- EP-A- 1 182 184
- WO-A-02/02504
- DE-A- 2 345 233
- DE-A- 3 714 602
- GB-A- 2 127 013

## Beschreibung

Die vorliegende Erfindung betrifft ein Verfahren zur Herstellung von halogensubstituierten Dibenzylalkoholen aus den entsprechenden halogensubstituierten Terephthalsäuren, die dadurch erhältlichen halogensubstituierten Dibenzylalkohole sowie deren Verwendung zur Herstellung von pharmazeutischen und agrochemischen Wirkstoffen.

2,3,5,6-Tetrahalogendibenzylalkohole sind dem Fachmann als wichtige Zwischenprodukte bekannt, beispielsweise zur Herstellung von pharmazeutischen oder agrochemischen Wirkstoffen wie Insektiziden. Insbesondere 2,3,5,6-Tetrafluordibenzylalkohol ist ein wichtiges Zwischenprodukt zur Herstellung von insektizid wirksamen Pyrethroiden.

Der Einsatz von 2,3,5,6-Tetrafluordibenzylalkohol als Einsatzkomponente zur Herstellung von Haushaltsinsektiziden ist beschrieben in z.B. EP-A-0 963 976, EP-A-0 959 065, EP-A-0 302 612 und EP-A-0 926 129.

Die Herstellung von 2,3,5,6-Tetrafluordibenzylalkohol durch Boranatreduktion aus Derivaten der Tetrafluorterephthalsäure ist beschrieben in GB-A-2,127,013. Hier wird die Reduktion von Tetrafluorterephthalsäurechlorid mit Natriumborhydrid in Diglyme bei einer Temperatur von 20°C zum Tetrafluordibenzylalkohol mit einer Ausbeute von lediglich 64,4 % beschrieben. Angaben zur Reinheit werden nicht gemacht.

Weiterhin wird in WO-A-00/68173 die Synthese von Tetrafluordibenzylalkoholen durch katalytische Reduktion des entsprechenden Tetrafluorterephthalnitrils unter Durchlaufen der Zwischenstufen Tetrafluorterephthtalaldehyd-tetraalkylacetal und Tetrafluorterephthalaldehyd beschrieben. Im Fall der Herstellung von 2,3,5,6-Tetrafluordibenzylalkohol wird allerdings nur eine Reinheit von 93,4 % erzielt. Die Gesamtausbeute ausgehend von 2,3,5,6-Tetrafluorterephthalnitril beträgt 48 % (siehe dort Beispiele 15 und 18). Nach dem gleichen Verfahren wird auch die Synthese von 2,3,5,6-Tetrafluorbenzylalkohol beschrieben. Die hierbei in mehreren Beispielen beschriebenen Ausbeuten von ca. 80 % und Reinheiten von >99 % liegen somit deutlich höher als bei der Synthese des entsprechenden Dibenzylalkohols.

Weiterhin wird die Synthese von 2,3,5,6-Tetrafluordibenzylalkohol in der noch nicht veröffentlichen Deutschen Patentanmeldung mit dem Aktenzeichen 100 03 320 durch Hydrierung von 2,3,5,6-Tetrafluorterephthalnitril zu 2,3,5,6-Tetrafluordibenzylamin und anschließende Diazotierung und Verkochung des Amins beschrieben. Der hierdurch erhaltene 2,3,5,6-Tetrafluordibenzylalkohol verfügt über eine Reinheit von 74,5 %, die für bestimmte Anwendungen aber noch nicht völlig zufriedenstellend ist. Die Gesamtausbeute ausgehend von 2,3,5,6-Tetrafluorterephthalnitril beträgt gemäß Beispiel 52,1 %.

In der WO-A-00/68173 wird ein Verfahren beschrieben, in dem, vergleichbar mit dem Verfahren der Deutschen Patentanmeldung mit dem Aktenzeichen 100 03 320, der 2,3,5,6-Tetrafluorbenzylalkohol aus 2,3,5,6-Tetrafluorbenzonitril hergestellt wird. Die dabei erreichte Reinheit von 99,3 % und Ausbeute von 85,1 % liegt somit deutlich höher als bei der Synthese des entsprechenden 2,3,5,6-Tetrafluordibenzylalkohols.

In DE-A-37 14 602 wird ein Verfahren zur Herstellung von 2,3,5,6-Tetrafluorbenzylalkoholen, die in der Position 4 durch einen Methylrest substituiert sein können, durch Umsetzung der 2,3,5,6-Tetrafluorbenzoesäuren, die in der Position 4 durch einen Methylrest substituiert sein können, mit Natriumboranat und anschließend mit einem Alkylierungsmittel in Gegenwart eines Verdünnungsmittels bei Temperaturen zwischen -20°C und +150°C beschrieben. Die Reaktion der Tetrafluorbenzoesäuren wird bevorzugt bei 0 bis 30°C und die anschließende Umsetzung mit dem Alkylierungsmittel bevorzugt bei 0 bis 100°C durchgeführt, wobei pro mol Ausgangsverbindung 0,5 bis 0,9 mol eines Alkylierungsmittels eingesetzt werden. Das Verfahren liefert den 2,3,5,6-Tetrafluorbenzylalkohol mit einer Ausbeute von 96,7 % und einer Reinheit von 98 %.

Die schwefelsaure Hydrolyse von 2,3,5,6-Tetrafluorterephthalnitril zu 2,3,5,6-Tetrafluorterephthalsäure ist beschrieben in EP-A-0 749 409.

Wie dem Fachmann bekannt ist, gehen dem Einsatz von Insektiziden, vor allem Haushaltsinsektiziden, intensive toxikologische Studien voraus. Insofern ist es wünschenswert, die Wirkstoffe in definierter und möglichst reiner Form einsetzen zu können. Störend bei der Synthese von Insektiziden sind - insbesondere beim Einsatz des 2,3,5,6-Tetrafluordibenzylalkohols - die in Benzylstellung asymmetrisch substituierten Nebenkomponenten wie 1-Benzylalkohol-4-benzylamine, 1-Benzaldehyd-4-benzylalkohole und 1-Benzoesäure-4-benzylalkohole, da diese Verbindungen über den Verlauf der Gesamtsynthese bis in die Endstufe gelangen, somit im Wirkstoff verbleiben und unerwünschte toxische Effekte ausüben können.

Alle drei vorgenannten Verfahren zur Herstellung von 2,3,5,6-Tetrafluordibenzylalkoholen (GB-A-2,127,013, WO-A-00/68173 und DE-A-100 03 320) haben den gemeinsamen Nachteil, dass mehr als eine Zwischenstufe ausgehend vom Tetrafluorterephtalnitril durchlaufen wird. Als entscheidender Nachteil kommt hinzu, dass bei allen anderen Verfahren, die sowohl für die Herstellung von Monobenzyl- als auch Dibenzylalkoholen eingesetzt werden, die Dibenzylverbindung jeweils nur mit einer deutlich schlechteren Ausbeute und Reinheit erhalten wird. Hieraus war zu schlussfolgern, dass offensichtlich die Dibenzylfunktion des gewünschten Produktes und die bei einer Synthese intermediär auftretenden asymmetrischen Zwischenprodukte sowohl die Reinheit als auch die Ausbeute stark beeinflussen.

Die Aufgabe der vorliegenden Erfindung bestand somit darin, ein Verfahren bereitzustellen, welches die Herstellung von halogensubstituierten Dibenzylalkoholen ausgehend von den entsprechenden halogensubstituierten Terephthalsäuren mit hohen Ausbeuten und Reinheiten und insbesondere weitestgehend frei von in 1- und 4-Stellung asymmetrisch substituierten Verbindungen ermöglicht.

Gegenstand der Erfindung ist ein Verfahren zur Herstellung von halogensubstituierten Dibenzylalkoholen der allgemeinen Formel (I), 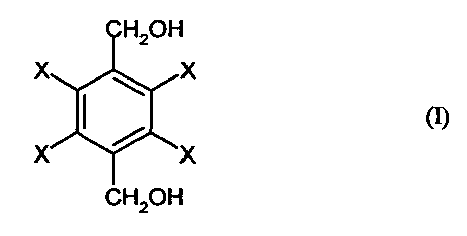 wobei X gleich oder verschieden ist und für Wasserstoff, Fluor, Chlor oder Brom steht und mindestens ein X Fluor, Chlor oder Brom bedeutet,
durch Umsetzung von halogensubstituierten Terephthalsäuren der allgemeinen Formel (II), 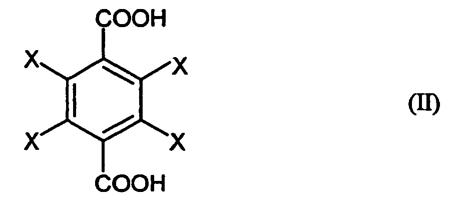 wobei X die für die allgemeine Formel (I) genannten Bedeutungen hat,
mit Natriumboranat und anschließend mit einem Alkylierungsmittel oder Schwefelsäure oder Alkyl- oder Arylsulfonsäuren, wobei die Schwefelsäure bzw. die Alkyl-oder Arylsulfonsäuren maximal 5 Vol.-% Wasser enthalten und die Umsetzung mit Natriumboranat in einem organischen Lösungsmittel bei einer Temperatur im Bereich von 0 bis 150°C durchgeführt wird.

Die erfindungsgemäße Boranatreduktion der halogensubstituierten Terephthalsäuren der allgemeinen Formel (II) unter den genannten Bedingungen und die anschließende Umsetzung mit Alkylierungsmittel, Schwefelsäure oder Alkyl- oder Arylsulfonsäure gelingt mit einer unerwartet hohen Reinheit bei gleichzeitig hohen Ausbeuten. Die erhaltenen Produkte der Formel (I) sind weitestgehend frei bzw. im bevorzugten Fall völlig frei von in 1- und 4-Stellung asymmetrisch substituierten Verbindungen, insbesondere halogensubstituierten 1-Benzylalkohol-4-benzylaminen, halogensubstituierten 1-Benzaldehyd-4-benzylalkoholen und halogensubstituierten 1-Benzoesäure-4-benzyl-alkoholen.

Ausgangsstoffe für das erfindungsgemäße Verfahren sind halogensubstituierte Terephthalsäuren der allgemeinen Formel (II), wobei X gleich oder verschieden ist und Wasserstoff, Fluor, Chlor oder Brom bedeutet und mindestens ein X Fluor, Chlor oder Brom darstellt. Bevorzugt werden 2,3,5,6-Tetrafluor- und 2,3,5,6-Tetrachlorterephthalsäure eingesetzt, d.h. alle vier Substituenten X sind gleich Fluor oder gleich Chlor. Auch der Einsatz von gemischt substituierten Terephthalsäuren, bei denen zwei X für Wasserstoff und je ein X für Chlor bzw. Fluor stehen, hat sich bewährt.

Die Herstellung derartiger halogensubstituierter Terephthalsäuren der allgemeinen Formel (I) ist dem Fachmann geläufig. Bevorzugt ist es, die halogenierten Terephthalsäuren der allgemeinen Formel (II) durch saure Hydrolyse von halogeniertem Terephthalnitril der allgemeinen Formel (III), 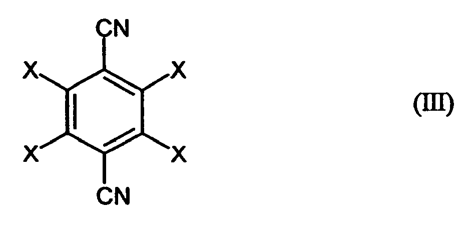 wobei X die gleiche Bedeutung hat wie in der allgemeinen Formel (III), herzustellen.

Diese saure Hydrolyse ist in einfacher Weise und mit hohen Reinheiten zu realisieren. Wird dieser Weg zur Herstellung der halogensubstituierten Terephthalsäure gewählt, so ist der durch das erfindungsgemäße Verfahren anschließend erhaltene halogenierte Dibenzylalkohol sehr rein. Besonders bewährt hat sich diese Variante zur Herstellung von 2,3,5,6-Tetrafluorterephthalsäure aus 2,3,5,6-Tetrafluorterephthalnitril.

Die Umsetzung mit Natriumboranat wird im erfindungsgemäßen Verfahren bei einer Temperatur im Bereich von 0 bis 150°C, bevorzugt 20 bis 80°C und insbesondere 40 bis 65°C durchgeführt.

Die erfmdungsgemäße Umsetzung mit Natriumboranat wird in einem organischen Lösungsmittel durchgeführt. Bevorzugt wird in einem aprotischem polaren organischen Lösungsmittel, wie z.B. Diethylether, Diisopropylether, Diisobutylether, Methyl-t-butylether, 1,2-Dimethoxyethan, Diglycoldimethylether, Tetrahydrofuran und 1,4-Dioxan gearbeitet. Besonders bevorzugt ist 1,2-Dimethoxyethan. In einer besonderen Ausführungsfonn wird Natriumboranat vorgelegt und die 2,3,5,6-Tetrafluorterephthalsäure als Lösung in diesem Lösungsmittel hinzugefügt.

Wird anschließend an die Boranatreduktion ein Alkylierungsmittel zugegeben, so hat es sich bewährt, für die Reduktion 2,0 bis 2,5 mol, bevorzugt 2,1 bis 2,4 mol, besonders bevorzugt 2,1 bis 2,3 mol Natriumboranat pro mol halogenierter Terephthalsäure der allgemeinen Formel (II) einzusetzen.

Wird dagegen anschließend an die Boranatreduktion Schwefelsäure oder eine Alkyl- bzw. Arylsulfonsäure eingesetzt, so hat es sich bewährt, für die Reduktion 2,0 bis 4,0 mol und bevorzugt 2,5 bis 3,0 mol Natriumboranat pro mol halogenierter Terephthalsäure der allgemeinen Formel (II) zu verwenden.

Als Alkylierungsmittel können beispielsweise Alkylhalogenide, Dialkylsulfate und Sulfonsäureester der allgemeinen Formeln (IV), (V) und (VI) eingesetzt werden, 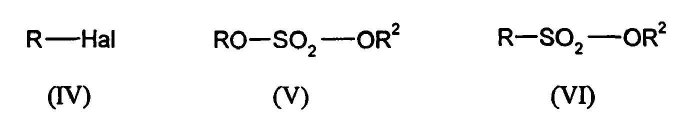 wobei R und R² unabhängig voneinander für Methyl, Ethyl, Propyl, iso-Propyl, n-Butyl, iso-Butyl, tert.-Butyl, Phenyl oder Tolyl stehen und Hal für Cl, Br oder I steht.

Dabei setzt man pro mol der ursprünglich eingesetzten halogenierten Terephthalsäure der allgemeinen Formel (II) 1,0 bis 2,0 mol und bevorzugt 1,4 bis 2,0 mol des Alkylierungsmittels ein.

Weiterhin ist es im erfindungsgemäße Verfahren möglich, dass man statt eines Alkylierungsmittels auch Schwefelsäure oder eine Alkyl- oder Arylsulfonsäure einsetzt, die maximal 5 Vol.-% Wasser enthält. Als Alkylsulfonsäure kann beispielsweise Methyl-, Ethyl-, Propyl- oder Butylsulfonsäure eingesetzt werden. Als Arylsulfonsäure hat sich Phenyl- oder Tolylsulfonsäure bewährt. Üblicherweise verwendet man in diesem Fall pro mol der ursprünglich eingesetzten halogenierten Terephthalsäure der allgemeinen Formel (II) 0,5 bis 1,0 mol Schwefelsäure oder 1,0 bis 2,0 mol einer Alkyl- oder Arylsulfonsäure, wobei Schwefelsäure bzw. Alkyl- oder Arylsulfonsäure jeweils maximal 5 Vol.-% Wasser enthalten.

Auch das Alkylierungsmittel kann in einem inerten Lösungsmittel verdünnt zugegeben werden. Hierbei können zum einen die polaren aprotischen organischen Lösungsmittel verwendet werden, die für die Boranatreduktion eingesetzt werden können. Als andere inerte Lösungsmittel zur Verdünnung des Alkylierungsmittels können C₅-C₃₀-, bevorzugt C₆-C₁₂-Alkane, C₅-C₃₀-, bevorzugt C₆-C₁₂-Cycloalkane, C₆₋C₂₀-, bevorzugt C₆-C₁₂-Aromaten, C₂-C₁₀-Ester und C₂-C₃₀-Ether geeignet. Bevorzugt werden Hexan, Heptan, Petrolether, Cyclohexan, Decalin, Benzol, Toluol, die Xylole, Chlorbenzol, Dichlorbenzol, Trichlorbenzol, Essigester, Butylacetat, Diethylether, Tetrahydrofuran und Diphenylether eingesetzt werden. Besonders bevorzugt sind Toluol und Xylol.

Die Umsetzung mit dem Alkylierungsmittel, der Schwefelsäure oder der Alkyl- bzw. Arylsulfonsäure wird im erfindungsgemäßen Verfahren beispielsweise bei Temperaturen im Bereich von 0 bis 150°C, bevorzugt 30 bis 80°C, besonders bevorzugt 40 bis 70°C und insbesondere 50 bis 65°C durchführt.

Das erfindungsgemäße Verfahren wird üblicherweise bei Normaldruck oder einem erhöhtem Druck von bis zu etwa 10 bar durchgeführt.

Die Aufarbeitung und Isolierung des Reaktionsproduktes kann nach den üblichen Methoden erfolgen. Vorzugsweise wird das Reaktionsgemisch mit einem mit Wasser praktisch nicht mischbaren Lösungsmittel verdünnt. Vorteilhaft ist auch die Möglichkeit des destillativen Lösungsmittelaustausches. Besonders vorteilhaft ist der Einsatz des Verdünnungsmittels des Alkylierungsmittels für diesen Zweck. Danach kann mit Wasser oder einer wässrigen Säure hydrolysiert werden. Die wässrige Phase kann extrahiert werden, gegebenenfalls bei höherer Temperatur, und aus dem Extraktionsmittel kann anschließend, z.B. nach Einengen oder wiederum einem Lösungsmitteltausch, um die Löslichkeit des Produktes im Lösungsmittel herabzusetzen, kristallisiert und durch Filtration isoliert werden.

Bei den Aufarbeitungsschritten, wie z.B. der Destillation, hat es sich bewährt, den Druck auf bis zu 0,001 bar abzusenken.

Das erfindungsgemäße Verfahren liefert über die einfachen Verfahrensschritte einer Boranatreduktion sowie Umsetzung mit Alkylierungsmittel, Schwefelsäure, Alkyl-oder Arylsulfonsäure den gewünschten halogenierten Dibenzylalkohol der allgemeinen Formel (I) in einer Reinheit von über 98,5 % bei gleichzeitig exzellent hohen Ausbeuten.

Die halogensubstituierten Dibenzylalkohole der allgemeinen Formel (I), die nach dem erfindungsgemäßen Verfahren erhältlich sind, besitzen eine Reinheit von mindestens 98,5 %. Sie enthalten maximal 1,0 %, bevorzugt maximal 0,1 %, besonders bevorzugt maximal 0,01 % und insbesondere sind sie völlig frei von in 1- und 4-Stellung asymmetrisch substituierten Verbindungen, insbesondere halogensubstituierten 1-Benzylalkohol-4-benzylaminen, 1-Benzaldehyd-4-benzylalkoholen und 1-Benzoesäure-4-benzylalkoholen. Der zur Ergänzung auf 100 erforderliche Anteil ist auf sonstige Verunreinigungen zurückzuführen, die in den Vorstufen zu den halogensubstituierten Terephthalsäuren über die Edukte mit ins System gebracht und bis zum Endprodukt mitgeschleppt werden.

Diese besonders reinen halogensubstituierten Dibenzylalkohole eignen sich als Zwischenprodukte zur Herstellung von pharmazeutischen und agrochemischen Wirkstoffen.

Hierbei kann sich der Fachmann an der umfangreichen Literatur beispielsweise zur Herstellung von Haushaltsinsektiziden orientieren, beispielsweise an EP-A-0 963 976, EP-A-0 959 065, EP-A-0 302 612 und EP-A-0 926 129.

### Beispiele

### Beispiel 1:

### Saure Hydrolyse von 2,3,5,6-Tetrafluorterephthalsäure

7320 g 98 %ige Schwefelsäure werden zusammen mit 30 g Natronwasserglas und 30 g Silikonöl vorgelegt und auf 100°C aufgeheizt. Hierzu werden in 6 Portionen je 254,2 g Tetrafluorterephthalnitril gegeben. Danach wird der Ansatz auf 170°C aufgeheizt und eine Stunde bei dieser Temperatur gerührt. Bei 170°C werden 2440 g Wasser innerhalb von 3 h zudosiert. Anschließend lässt man auf 20°C abkühlen und filtriert den ausgefallenen Feststoff ab. Der Rückstand wird zweimal mit je 500 g Wasser nachgewaschen und getrocknet. Man erhält 1769,0 g Tetrafluorterephthalsäure mit einem Gehalt von 96 % (Rest zu 100 % anorganische Bestandteile der Silikate) (99,5 % Ausbeute).

### Beispiel 2:

### Boranatreduktion mit Dimethylsulfat von 2,3,5,6-Tetrafluorterephthalsäure zu 2,3,5,6-Tetrafluordibenzylalkohol

In einem 16 Liter Glasreaktor werden 534,5 g Natriumborhydrid und 3000 g 1,2-Dimethoxyethan vorgelegt. Der Reaktorinhalt wird für 3 h bei 60°C unter Stickstoffatmosphäre gerührt. Daraufhin wird bei 60°C innerhalb von 10 h 4200 g einer 33,3 Gew.-% Lösung von 2,3,5,6-Tetrafluorterephthalsäure in 1,2-Dimethoxyethan zudosiert (die Lösung wird vor dem Einsatz von anorganischen Bestandteilen aus der Hydrolyse durch Filtration befreit). Der Reaktorinhalt wird bei 60°C gerührt bis die Gasentwicklung beendet ist. Es wird auf 50°C abgekühlt und anschließend 2000 g Toluol hinzugefügt. Anschließend wird eine 21,5 Gew.-% Lösung aus 1480,9 g Dimethylsulfat und 5400 g Toluol innerhalb von 10 h bei 50°C hinzugefügt. Der Reaktorinhalt wird bei 50°C gerührt bis die Gasentwicklung beendet ist. Anschließend wird der Reaktionsansatz 1 h bei 75°C und daraufhin auf Rückfluss erhitzt. Durch Zugabe von Toluol unter gleichzeitigem Abdestillieren von 1,2-Dimethoxyethan wird das Lösungsmittel ausgetauscht.

Die Destillation ist beendet, wenn der Reaktorinhalt auf ca. 8 Liter eindestilliert ist und nahezu kein 1,2-Dimethoxyethan in der Destillation mehr mit übergeht.

Es wird auf 65°C abgekühlt und innerhalb von 2 h 4500 g Wasser hinzugefügt. Der Ansatz wird auf 90°C erhitzt und für 10 h gerührt. Danach wird auf 60°C abgekühlt und 1500 g Essigester hinzugefügt und für 0,5 h gerührt. Die Phasen werden getrennt. Die wässrige Phase wird zweimal mit je 5000 g Essigester extrahiert. Die vereinigten organischen Phasen werden auf 12 Liter eingeengt, wobei eventuell noch einmal 3000 g Toluol gegen Essigester destillativ ausgetauscht werden. Bei 80°C wird mit 500 g gesättigter Natriumcarbonat-Lösung gewaschen und anschließend auf 20°C abgekühlt. Der ausgefallene Feststoff wird durch Filtration isoliert und gegebenenfalls mit 1000 g Toluol gewaschen, um die anhaftende Mutterlauge zu verdrängen. Das Produkt wird getrocknet. Man erhält 1123,5 g (90,5 %) eines farblosen Feststoffes mit einer Reinheit von 98,7 %.

### Beispiel 3:

### Boranatreduktion mit Schwefelsäure von 2,3,5,6-Tetrafluorterephthalsäure zu 2,3,5,6-Tetrafluordibenzylalkohol

In einem 16 Liter Glasreaktor werden 617,5 g Natriumborhydrid und 1950 g 1,2-Dimethoxyethan vorgelegt. Daraufhin wird bei 60°C innerhalb von 10 h 3900 g einer 31,5 Gew.-% Lösung von 2,3,5,6-Tetrafluorterephthalsäure in 1,2-Dimethoxyethan zudosiert. Der Reaktorinhalt wird bei 60°C gerührt bis die Gasentwicklung beendet ist. Es wird auf 50°C abgekühlt und anschließend 2000 g Toluol hinzugefügt. Anschließend werden simultan 793 g 98 %ige Schwefelsäure und 1549 g Toluol innerhalb von 10 h bei 50°C hinzugefügt. Der Reaktorinhalt wird bei 65°C gerührt bis die Gasentwicklung beendet ist. Durch Zugabe von Toluol unter gleichzeitigem Abdestillieren von 1,2-Dimethoxyethan wird das Lösungsmittel ausgetauscht.

Die Destillation ist beendet, wenn der Reaktorinhalt auf ca. 8 Liter eindestilliert ist und nahezu kein 1,2-Dimethoxyethan in der Destillation mehr mit übergeht.

Es wird auf 65°C abgekühlt und innerhalb von 2 h 3640 g 5 %ige Natronlauge hinzugefügt. Der Ansatz wird auf 90°C erhitzt und für 3 h gerührt. Danach wird auf 60°C abgekühlt und 1500 g Essigester und 500 g Wasser hinzugefügt und für 0,5 h gerührt. Die Phasen werden getrennt. Die wässrige Phase wird einmal mit 4550 g Essigester extrahiert. Die vereinigten organischen Phasen werden auf 6 Liter eingeengt und anschließend auf 20 °C abgekühlt. Der ausgefallene Feststoff wird durch Filtration isoliert und gegebenenfalls mit 650 g Toluol gewaschen, um die anhaftende Mutterlauge zu verdrängen. Das Produkt wird getrocknet. Man erhält 661,2 g (60,4 %) eines farblosen Feststoffes mit einer Reinheit von 99,1%.

### Vergleichsbeispiele

### 1. Herstellung des 2,3,5,6-Tetrafluor-xylylidendiamins als schwefelsaure Lösung

1249,5 g 2,3,5,6-Tetrafluorterephthalsäuredinitril (6,24 mol) werden zusammen mit 750 ml Methanol, 5250 ml Wasser, 1050 g konzentrierter Schwefelsäure und 75 g eines 5 %igen Palladium-Kohle Katalysators in einem 10 1 Autoklav vorgelegt und bei 30°C und 30 bar Wasserstoffdruck bis zur Druckkonstanz hydriert. Der Katalysator wird abfiltriert. Anschließend wird das Methanol bei 80 bis 90°C und 350 mbar wieder abdestilliert. Man erhält 6690 g einer schwefelsauren Lösung, die nach GC Analyse (externer Standard) 1197,2 g (5,75 mol) Tetrafluor-xylylidendiamin (92 % Ausbeute) enthält.

### 2. Herstellung des 2,3,5,6-Tetrafluordibenzylalkohols

Von einer wie oben unter 1. hergestellten Lösung Tetrafluorxylylidendiamin Hydrosulfat werden 3200 g (2,87 mol Verbindung) mit 868 g 20 %iger NatriumhydroxidLösung auf den pH 4 eingestellt. Die Reaktionsmischung wird auf 80 bis 90°C erhitzt und lässt innerhalb von 4 h 981 g einer 40 %igen Natriumnitrit-Lösung zutropfen. Gleichzeitig hält man den pH-Wert der Lösung mit Hilfe von 127 g 30 %iger Schwefelsäure zwischen pH 3 und pH 5. Nach beendeter Gasentwicklung wird der Reaktionsansatz zweimal mit 750 ml Ethylacetat extrahiert und die organische Phase anschließend im Vakuum eingeengt. Man erhält 456 g (2,17 mol) 2,3,5,6-Tetrafluorxylylidendiol in einer Reinheit von 74,5 % und einer Ausbeute von 76 %.

## Patentansprüche

1. Verfahren zur Herstellung von halogensubstituierten Dibenzylalkoholen der allgemeinen Formel (I), 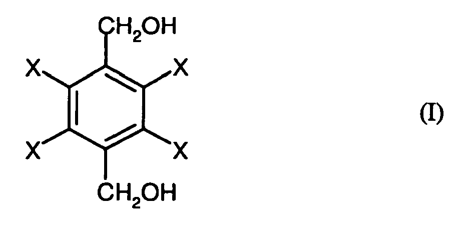 wobei X gleich oder verschieden ist und für Wasserstoff, Fluor, Chlor oder Brom steht und mindestens ein X Fluor, Chlor oder Brom bedeutet,
durch Umsetzung von halogensubstituierten Terephthalsäuren der allgemeinen Formel (II), 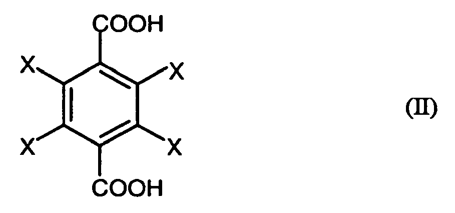 wobei X die für die allgemeine Formel (I) genannten Bedeutungen hat,
mit Natriumboranat und anschließend mit einem Alkylierungsmittel oder Schwefelsäure oder Alkyl- oder Arylsulfonsäuren, wobei die Schwefelsäure bzw. die Alkyl- oder Arylsulfonsäuren maximal 5 Vol.-% Wasser enthalten und die Umsetzung mit Natriumboranat in einem organischen Lösungsmittel bei einer Temperatur im Bereich von 0 bis 150 °C durchgeführt wird.

2. Verfahren nach Anspruch 1, **dadurch gekennzeichnet, dass** 2,3,5,6-Tetrafluor- oder 2,3,5,6-Tetrachlorterephthalsäure als halogenierte Terephthalsäuren der allgemeinen Formel (II) eingesetzt werden.

3. Verfahren nach Anspruch 1, **dadurch gekennzeichnet, dass** halogenierte Terephthalsäuren der allgemeinen Formel (II) eingesetzt werden, bei denen zwei X für Wasserstoff und je ein X für Chlor bzw. Fluor stehen.

4. Verfahren nach einem oder mehreren der Ansprüche 1 bis 3, **dadurch gekennzeichnet, dass** die halogenierten Terephthalsäuren der allgemeinen Formel (II) durch saure Hydrolyse von halogeniertem Terephthalnitril der allgemeinen Formel (III), 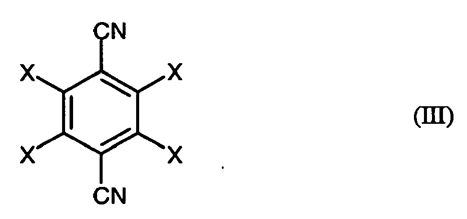 wobei X die gleiche Bedeutung hat wie in der allgemeinen Formel (III), erhalten werden.

5. Verfahren nach einem oder mehreren der Ansprüche 1 bis 4, **dadurch gekennzeichnet, dass** die Umsetzung mit Natriumboranat bei einer Temperatur im Bereich von 20 bis 80°C und bevorzugt 40 bis 65°C durchgeführt wird.

6. Verfahren nach einem oder mehreren der Ansprüche 1 bis 5, **dadurch gekennzeichnet, dass** es sich bei dem organischen Lösungsmittel um ein aprotisch polares organisches Lösungsmittel handelt, bevorzugt um Diethylether, Diisopropylether, Diisobutylether, Methyl-t-butylether, 1,2-Dimethoxyethan, Diglycoldimethylether, Tetrahydrofuran oder 1,4-Dioxan.

7. Verfahren nach einem oder mehreren der Ansprüche 1 bis 6, **dadurch gekennzeichnet, dass** 2,0 bis 2,5 mol, bevorzugt 2,1 bis 2,4 mol, besonders bevorzugt 2,1 bis 2,3 mol Natriumboranat pro mol halogenierter Terephthalsäure der allgemeinen Formel (II) eingesetzt werden und anschließend ein Alkylierungsmittel zugesetzt wird.

8. Verfahren nach einem oder mehreren der Ansprüche 1 bis 6, **dadurch gekennzeichnet, dass** 2,0 bis 4,0 mol und bevorzugt 2,5 bis 3,0 mol Natriumboranat pro mol halogenierter Terephthalsäure der allgemeinen Formel (II) eingesetzt werden und anschließend Schwefelsäure oder eine Alkyl- bzw. Arylsulfonsäure zugesetzt werden.

9. Verfahren nach einem oder mehreren der Ansprüche 1 bis 7, **dadurch gekennzeichnet, dass** als Alkylierungsmittel Alkylhalogenide, Dialkylsulfate oder Sulfonsäureester der allgemeinen Formeln (III), (IV) und (V) eingesetzt werden, 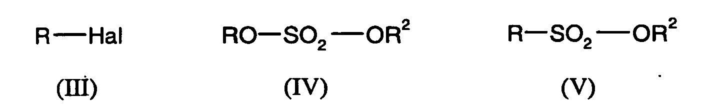 wobei R und R² unabhängig voneinander für Methyl, Ethyl, Propyl, iso-Propyl, n-Butyl, iso-Butyl, tert.-Butyl, Phenyl oder Tolyl stehen und Hal für Cl, Br oder I steht.

10. Verfahren nach einem oder mehreren der Ansprüche 1 bis 8, **dadurch gekennzeichnet, dass** als Alkylsulfonsäure Methyl-, Ethyl-, Propyl- oder Butylsulfonsäure eingesetzt werden oder als Arylsulfonsäure Phenyl- oder Tolylsulfonsäure.

11. Verfahren nach einem oder mehreren der Ansprüche 1 bis 10, **dadurch gekennzeichnet, dass** die Umsetzung mit dem Alkylierungsmittel, der Schwefelsäure oder der Alkyl- bzw. Arylsulfonsäure bei einer Temperatur im Bereich von 0 bis 150°C, bevorzugt 30 bis 80°C, besonders bevorzugt 40 bis 70°C und insbesondere 50 bis 65°C durchgeführt wird.

## Claims

1. Process for preparing halogen-substituted dibenzyl alcohols of the general formula (I), 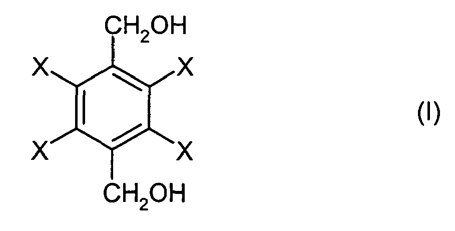 where the radicals X are identical or different and are hydrogen, fluorine, chlorine or bromine and at least one X is fluorine, chlorine or bromine,
by reacting halogen-substituted terephthalic acids of the general formula (II), 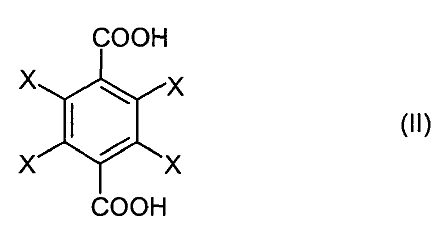 where the radicals X are as defined for the general formula (I),
with sodium borohydride and then with an alkylating agent or sulphuric acid or alkyl- or arylsulphonic acids, where the sulphuric acid or the alkyl-or arylsulphonic acids comprise at most 5% by volume of water and the reaction with sodium borohydride is carried out in an organic solvent at a temperature in the range from 0 to 150°C.

2. Process according to Claim 1, **characterized in that** the halogenated terephthalic acid of the general formula (II) used is 2,3,5,6-tetrafluoro- or 2,3,5,6-tetrachloroterephthalic acid.

3. Process according to Claim 1, **characterized in that** halogenated terephthalic acids of the general formula (II) are used in which two X are hydrogen and in each case one X is chlorine or fluorine.

4. Process according to one or more of Claims 1 to 3, **characterized in that** the halogenated terephthalic acids of the general formula (II) are obtained by acid hydrolysis of halogenated terephthalonitrile of the general formula (III), 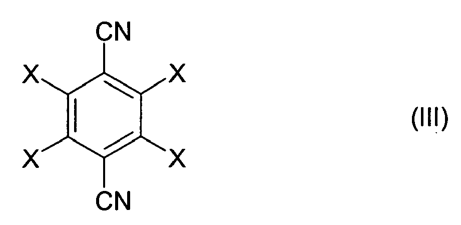 where the radicals X are as defined for the general formula (III).

5. Process according to one or more of Claims 1 to 4, **characterized in that** the reaction with sodium borohydride is carried out at a temperature in the range from 20 to 80°C and preferably 40 to 65°C.

6. Process according to one or more of Claims 1 to 5, **characterized in that** the organic solvent is an aprotic polar organic solvent, preferably diethyl ether, diisopropyl ether, diisobutyl ether, methyl t-butyl ether, 1,2-dimethoxyethane, diglycol dimethyl ether, tetrahydrofuran or 1,4-dioxane.

7. Process according to one or more of Claims 1 to 6, **characterized in that** 2.0 to 2.5 mol, preferably 2.1 to 2.4 mol, particularly preferably 2.1 to 2.3 mol of sodium borohydride are employed per mole of halogenated terephthalic acid of the general formula (II) and an alkylating agent is then added.

8. Process according to one or more of Claims 1 to 6, **characterized in that** 2.0 to 4.0 mol and preferably 2.5 to 3.0 mol of sodium borohydride are employed per mole of halogenated terephthalic acid of the general formula (II) and sulphuric acid or an alkyl- or arylsulphonic acid is then added.

9. Process according to one or more of Claims 1 to 7, **characterized in that** the alkylating agent used is an alkyl halide, dialkyl sulphate or sulphonic ester of the general formula (III), (IV) or (V), 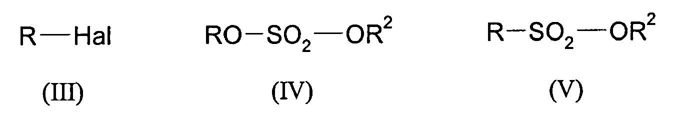 where R and R² independently of one another are methyl, ethyl, propyl, isopropyl, n-butyl, isobutyl, tert-butyl, phenyl or tolyl and Hal is Cl, Br or I.

10. Process according to one or more of Claims 1 to 8, **characterized in that** the alkylsulphonic acid used is methyl-, ethyl-, propyl- or butylsulphonic acid or the arylsulphonic acid used is phenyl- or tolylsulphonic acid.

11. Process according to one or more of Claims 1 to 10, **characterized in that** the reaction with the alkylating agent, the sulphuric acid or the alkyl- or arylsulphonic acid is carried out at a temperature in the range from 0 to 150°C, preferably 30 to 80°C, particularly preferably 40 to 70°C and especially 50 to 65°C.

## Revendications

1. Procédé de préparation d'alcools dibenzyliques halogéno-substitués de formule générale (I), 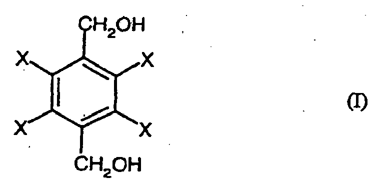 dans laquelle X est identique ou différent et représente un atome d'hydrogène, un atome de fluore, un atome de chlore ou un atome de brome, et au moins un X représente un atome de fluore, un atome de chlore ou un atome de brome,
par réaction d'acides téréphtaliques halogéno-substitués de formule générale (II), 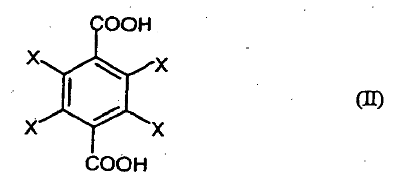 dans laquelle X présente les significations mentionnées pour la formule générale (I),
avec du boranate de sodium et ensuite avec un agent d'alkylation ou de l'acide sulfurique, ou des acides alkylsulfoniques ou arylsulfoniques, où l'acide sulfurique ou les acides alkylsulfoniques ou arylsulfoniques contiennent au maximum 5 % en volume d'eau, et la réaction avec le boranate de sodium est effectuée dans un solvant organique à une température dans la plage de 0 à 150°C.

2. Procédé selon la revendication 1, **caractérisé en ce que** l'on utilise, en tant qu'acides téréphtaliques halogénés de formule générale (II), de l'acide 2,3,5,6-tétrafluorotéréphtalique ou de l'acide 2,3,5,6-tétrachlorotéréphtalique:

3. Procédé selon la revendication 1, **caractérisé en ce que** l'on utilise des acides téréphtaliques halogénés de formule générale (II), dans lesquels deux X représentent un atome d'hydrogène et chaque X représente un atome de chlore ou un atome de fluore.

4. Procédé selon l'une ou plusieurs des revendications 1 à 3, **caractérisé en ce que** les acides téréphtaliques halogénés de formule générale (II) sont obtenus par hydrolyse acide d'un téréphtalonitrile halogéné de formule générale (III), 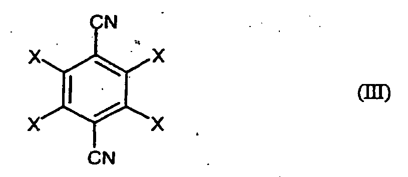 dans laquelle X présente la même signification que dans la formule générale (III).

5. Procédé selon l'une ou plusieurs des revendications 1 à 4, **caractérisé en ce que** la réaction avec du boranate de sodium est effectuée à une température dans la plage de 20 à 80°C et de préférence de 40 à 65°C.

6. Procédé selon l'une ou plusieurs des revendications 1 à 5, **caractérisé en ce que** le solvant organique est un solvant organique polaire aprotique, de préférence l'éther diéthylique, l'éther diisopropylique, l'éther diisobutylique, l'éther méthyl-t-butylique, le 1,2-diméthoxyéthane, l'éther diméthylique de diglycol, le tétrahydrofurane ou le 1,4-dioxane.

7. Procédé selon l'une ou plusieurs des revendications 1 à 6, **caractérisé en ce que** de 2,0 à 2,5 mol, de préférence de 2,1 à 2,4 mol, particulièrement préférablement de 2,1 à 2,3 mol de boranate de sodium sont utilisées par mole d'acide téréphtalique halogéné de formule générale (II), et un agent d'alkylation est ensuite ajouté.

8. Procédé selon l'une ou plusieurs des revendications 1 à 6, **caractérisé en ce que** de 2,0 à 4,0 mol, et de préférence de 2,5 à 3,0 mol de boranate de sodium sont utilisées par mole d'acide téréphtalique halogéné de formule générale (II), et de l'acide sulfurique ou un acide alkylsulfonique ou arylsulfonique est ensuite ajouté.

9. Procédé selon l'une ou plusieurs des revendications 1 à 7, **caractérisé en ce que** l'on utilise, en tant qu'agent d'alkylation, des halogénures d'alkyle, des sulfates de dialkyle ou des esters d'acide sulfonique de formules générales (III), (IV) et (V), 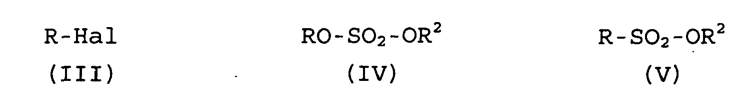 dans lesquelles R et R² représentent, indépendamment l'un de l'autre, un groupe méthyle, un groupe éthyle, un groupe propyle, un groupe isopropyle, un groupe n-butyle, un groupe isobutyle, un groupe tert-butyle, un groupe phényle ou un groupe tolyle, et Hal représente un atome de chlore, un atome de brome ou un atome d'iode.

10. Procédé selon l'une ou plusieurs des revendications 1 à 8, **caractérisé en ce que** l'on utilise, en tant qu'acide alkylsulfonique, de l'acide méthylsulfonique, éthylsulfonique, propylsulfonique ou butylsulfonique, ou en tant qu'acide arylsulfonique, de l'acide phénylsulfonique ou tolylsulfonique.

11. Procédé selon l'une ou plusieurs des revendications 1 à 10, **caractérisé en ce que** la réaction avec l'agent d'alkylation, l'acide sulfurique ou l'acide alkylsulfonique ou arylsulfonique est effectuée à une température dans la plage de 0 à 150°C, de préférence de 30 à 80°C, particulièrement préférablement de 40 à 70°C, et en particulier de 50 à 65°C.
